# EUROPÄISCHE PATENTANMELDUNG

(11) **EP 4 806 484 A1**
(43) Veröffentlichungstag der Anmeldung: **16.09.2026**
(21) Anmeldenummer: 25163872.2
(22) Anmeldetag: 14.03.2025
(51) Int. Cl.: A61M 15/08, A61F 9/00, B05B 11/00

(54) **MEDIZINISCHER SPENDER**

(71) Anmelder: Aptar Radolfzell GmbH, 78315 Radolfzell (DE)
(72) Erfinder: Greiner-Perth, Jürgen, 78244 Gottmadingen (DE)
(74) Vertreter: Witte, Weller und Partner Patentanwälte mbB Stuttgart

(57) **Zusammenfassung**

Bekannt ist sind kleine medizinischer Flüssigkeitsspender mit einer Länge von maximal 125 mm und üblicherweise weniger als 20 Gramm Leergewicht, beispielsweise in Form von Nasalspendern oder Augentropenspendern.

Um die Recycling-Fähigkeit solcher Flüssigkeitsspender zu verbessern, werden Flüssigkeitsspender vorgeschlagen, die ausschließlich aus Kunststoffmaterial bestehen und deren Flüssigkeitsspeicher dahingehend optimiert ist, um bei modernen Recycling-Verfahren mit Objektsortierung auf Basis von gezielten Luftstößen richtig zugeordnet werden zu können.

Hierfür wird insbesondere vorgeschlagen, dass zur verbesserten Separierung im Rahmen des Abfallrecyclings ein Querschnitt des Flüssigkeitsspeichers flach ausgebildet ist, so dass eine liegende Position des Flüssigkeitsspenders auf einem Recyclingförderband in einer von zwei liegenden Ausrichtungen begünstigt wird.

## Beschreibung

Die Erfindung betrifft den Bereich medizinischer Flüssigkeitsspender und des Abfallrecyclings solcher Flüssigkeitsspender.

Das Recycling von Abfällen trägt wesentlich zum Umweltschutz bei, indem es Ressourcenverbrauch reduziert. Zunehmend bestehen gesetzliche Anforderungen und Anforderungen von Kundenseite in Hinblick auf die Recycling-Fähigkeit von Produkten. Solche Anforderungen betreffen neben der Frage, welche Materialien gemeinsam verwendet und recycelt werden können, auch die Frage, ob die Produkte im Rahmen einer automatisierten Trennung von anderweitigem Abfall aus anderem Material getrennt werden können, um sortenrein weiterverwertet zu werden.

In modernen Recyclinganlagen erfolgt die automatisierte Trennung von Kunststoffabfällen mittels eines Verfahrens, bei dem beispielsweise mittels eines Infrarot-Scanners die Materialzusammensetzung von Objekten des Abfalls auf einem Förderband anhand reflektierter Strahlung bestimmt wird. Parallel wird die genaue Position der Objekte auf dem Förderband bestimmt. Sobald die Kunststoffsorte erkannt wurde, steuern präzise Hochgeschwindigkeitsventile gezielte Luftstöße, die einzelne Objekte des Abfalls je nach Material gezielt in verschiedene Sammelbehälter lenken.

Es hat sich gezeigt, dass kleinere medizinische Flüssigkeitsspender bei Anwendung dieser Technik nicht zuverlässig in den richtigen Sammelbehälter gelangen. Es ist somit derzeit bei kleinen Flüssigkeitsspendern schwierig, den gesetzlichen Anforderungen oder Kundenanforderungen zu entsprechen.

### AUFGABE UND LÖSUNG

Aufgabe der Erfindung ist es, medizinische Flüssigkeitsspender im Hinblick auf eine hohe Recycling-fähigkeit zu verbessern.

Vorgeschlagen wird hierfür ein medizinischer Flüssigkeitsspender, der in gattungsüblicher Weise über einen Flüssigkeitsspeicher und einen daran angebrachten Austragkopf verfügt. Der Austragkopf und der Flüssigkeitsspeicher können beispielsweise über eine Schnappverbindung oder eine Gewindeverbindung verbunden sein. Gemeinsam bilden die Außenflächen des Austragkopfes und des Flüssigkeitsspeichers zumindest Teile der Außenkontur des Flüssigkeitsspenders und insbesondere die gesamte Außenkontur.

Erfindungsgemäße Flüssigkeitsspender verfügen im Lieferzustand üblicherweise über eine Kunststoffkappe, die eine Austragöffnung am Austragkopf bei Nichtgebrauch schützt. Wenn diese Kunststoffkappe zum Zeitpunkt des Recyclings noch vorhanden ist, so bildet sie einen Teil der Außenfläche des Austragkopfes und ist in der weiteren Beschreibung mit umfasst, wenn der Austragkopf genannt wird und nicht explizit darauf hingewiesen ist, dass es sich um den Austragkopf ohne Schutzkappe handelt.

Die hier beschriebenen Flüssigkeitsspender sind Spender einer besonders kleinen Art. Ihre Länge in einer Haupterstreckungsrichtung beträgt weniger als 125 mm, zumindest bei abgenommener Schutzkappe. Als Haupterstreckungsrichtung wird dabei die Erstreckungsrichtung vom Austragkopf zum Flüssigkeitsspeicher angesehen. Die Leermasse eines solchen kleinen Flüssigkeitsspenders beträgt 20 Gramm oder weniger, wobei zum Zeitpunkt des Recyclings noch Restflüssigkeit im Flüssigkeitsspeicher enthalten sein kann und die Masse geringfügig vergrößern kann. Vorzugsweise ist der Flüssigkeitsspender derart ausgestaltet, dass eine nicht entnehmbare Restflüssigkeitsmenge weniger als 3 ml beträgt.

Insbesondere noch kleinere oder leichtere Flüssigkeitsspender mit einer Länge von weniger als 100 mm und/oder einer Masse von weniger als 15 Gramm im entleerten Zustand können als Flüssigkeitsspender gemäß der vorliegenden Erfindung ausgestaltet sein.

Die hier beschriebenen Flüssigkeitsspender bestehen ausschließlich aus Kunststoffmaterial. Selbst geringe metallische Anteile, beispielsweise eine metallische Pumpenfeder oder Ventilkugel, sind bei der mit der Erfindung angestrebten Recycling-Qualität nicht akzeptabel.

Insbesondere können die Materialien des Flüssigkeitsspenders derart gewählt sein, dass alle Materialien in einem gemeinsamen Recycling-Stream verwertet werden können.

Zwei bevorzugte Ausgestaltungen sehen vor, dass der Spender ausschließlich aus Polyethylen (PE) oder ausschließlich aus Polypropylen (PP) besteht. Auch Gestaltungen mit einer Kombination aus PE und PP sind möglich. Eine weitere besonders bevorzugte Gestaltung sieht vor, dass der Spender ausschließlich aus Polyethylenterephthalat (PET) und Polyolefinen besteht, insbesondere aus PET und PP oder aus PET und PE. Insbesondere kann der Flüssigkeitsspeicher aus PET bestehen, während der Austragkopf zumindest zum Teil aus PE oder PP besteht. PET und PE bzw. PP können nach Zerkleinerung in einem nachfolgenden Recyclingschritt beispielsweise mittels Schwimmtrennung voneinander getrennt werden.

Erfindungsgemäße Flüssigkeitsspender können insbesondere mit einer Pumpeinrichtung versehen sein und am Austragkopf über eine gegenüber einer Basis des Austragkopfes bewegliche Betätigungseinheit verfügen, die zum Zwecke der Pumpbetätigung und zum Zwecke des Flüssigkeitsaustrags manuell niedergedrückt wird. Flüssigkeit wird hierdurch in Richtung einer Austragöffnung gedrückt und dort abgegeben, je nach Spender in Form eines unzerstäubten Strahls, eines zerstäubten Sprühstrahls oder in Form von Tropfen.

Insbesondere betrifft die Erfindung die folgenden Arten von kleinen Flüssigkeitsspendern:
Ein erfindungsgemäßer Flüssigkeitsspender kann insbesondere als Nasalspender ausgebildet sein. Er verfügt in einem solchen Fall am Austragkopf über einen länglichen Nasalapplikator, der sich vorzugsweise in der Haupterstreckungsrichtung des Flüssigkeitsspenders erstreckt. Am distalen Ende des Nasalapplikators ist eine Austragöffnung vorgesehen, die insbesondere für eine zerstäubte Flüssigkeitsabgabe vorgesehen sein kann. Beispielsweise kann hier zu diesem Zweck eine Wirbelkammer vorgesehen sein. Der Nasalapplikator wird bei der Benutzung in das Nasenloch des Benutzers eingeschoben, bevor durch Betätigung einer Pumpeinrichtung Flüssigkeit zur Austragöffnung gefördert und abgegeben wird.

Ein anderer Spendertyp, der bevorzugt in erfindungsgemäßer Weise ausgebildet ist, ist ein Tropfenspender, insbesondere zur Abgabe von Tropfen in ein Auge des Benutzers. Ein solcher Tropfenspender weist vorzugsweise eine Austragöffnung auf, die von einer Tropfenanlagefläche umgeben ist, an der sich bei bestimmungsgemäßer Betätigung des Spenders Flüssigkeit drucklos sammelt, um sich bei ausreichendem Volumen als Tropfen zu lösen. Der Austragdruck und die Tropfenabgabefläche sind derart aufeinander abgestimmt, dass durch die Austragöffnung ausgetragene Flüssigkeit nicht unmittelbar zu einem Abgabestrahl führt.

Ein weiterer Spendertyp, der vorzugsweise in erfindungsgemäßer Art ausgebildet ist, ist ein topischer Flüssigkeitsspender zur Abgabe von Flüssigkeit auf die Haut, insbesondere von höherviskoser Flüssigkeit in Art von Lotionen. Vorzugsweise ist bei solchen Spendern vorgesehen, dass die durch die Austragöffnung bediente Abgaberichtung mit der Haupterstreckungsrichtung des Spenders einen Winkel zwischen 45° und 135° einschließt, vorzugsweise einen rechten Winkel.

Nachfolgend werden verschiedene Maßnahmen genannt, die sich bei der Überwindung des eingangs genannten Problems der nicht ausreichend guten Trennbarkeit gattungsgemäßer Flüssigkeitsspender von anderweitigem Abfall als hilfreich erwiesen haben.

Die erfindungsgemäß wesentlichste Maßnahme besteht darin, einen Querschnitt des Flüssigkeitsspeichers im Bereich einer umlaufenden Speicherwandung flach auszubilden, also von der üblichen kreiszylindrischen Bauweise abzuweichen. Die flache Bauweise ist so zu gestalten, dass eine liegende Position auf einem Recyclingförderband in einer von zwei liegenden Ausrichtungen begünstigt wird. Der Flüssigkeitsspender weist demnach einen insgesamt abgeflachten Flüssigkeitsspeicher auf, der auf zwei gegenüberliegenden Seiten durch flache Wandungsabschnitte gebildet wird, von denen einer die Auflagefläche bildet, wenn der Flüssigkeitsspender auf einem Förderband gefördert wird. Unter einem flachen Wandungsabschnitt wird nicht zwingend ein ebener Wandungsabschnitt verstanden. Vielmehr sind die beiden flachen Wandungsabschnitte vorzugsweise leicht gewölbt, wobei ihr Krümmungsradius wesentlich größer ist als der Krümmungsradius in einem stark gekrümmten Randbereich, der die flachen Wandungsabschnitte verbindet.

Die flache Bauweise des Flüssigkeitsspeichers führt dazu, dass der Flüssigkeitsspender auf dem Förderband der Recyclinganlage in definierter Ausrichtung liegt. Eine stehende Ausrichtung ist aufgrund des Schwerpunktes des Flüssigkeitsspenders und der von der Kreisform abweichenden Speicherunterseite unwahrscheinlich. Die daher nahezu zwingend eintretende liegende Ausrichtung kann nur in zwei möglichen und gleichwertigen Ausrichtungen erfolgen. Jede andere Ausrichtung ist aufgrund der Formgebung des Flüssigkeitsspeichers höchst unwahrscheinlich. Die flach liegende Ausrichtung ist insbesondere auch für die nachfolgende Beaufschlagung mit einem Luftstoß von erheblicher Bedeutung, wie im Weiteren noch erläutert wird.

Die flache Bauweise führt weiterhin dazu, dass die Position des Flüssigkeitsspenders auf dem Förderband berechenbar bleibt. Es wurde festgestellt, dass trotz ruhigem Lauf des Förderbandes bei leichten und kleinen Flüssigkeitsspendern mit kreisrundem Querschnitt des Flüssigkeitsspeichers eine Rollbewegung eintreten kann und somit die für die Sortierung genutzten Luftstöße den Flüssigkeitsspender nicht zentral treffen, was deren Flugbahn negativ beeinflusst.

Insbesondere jedoch hat sich gezeigt, dass die definierte Ausrichtung des Flüssigkeitsspenders auf dem Förderband zusammen mit der vergleichsweise großen Anströmfläche, die der Flüssigkeitsspeicher durch seine beiden flachen Wandungsabschnitte bietet, eine erheblich verbesserte Beeinflussung der Flugbahn des Flüssigkeitsspenders ermöglicht.

Es wird angenommen, dass eines der Hauptprobleme beim Anströmen bekannter Flüssigkeitsspender darin liegt, dass der Luftstoß, der auf den runden Flüssigkeitsspeicher trifft, diesen primär mit einem Drall versieht. Die vom Luftstoß zur Verfügung gestellte Energie geht daher primär in eine rotierende oder gar trudelnde Bewegung des Flüssigkeitsspeichers über und weniger in die gewünschte Beeinflussung der Flugbahn. Der Flüssigkeitsspender mit rundem Flüssigkeitsspeicher erreicht daher trotz Luftstoß häufig nicht den richtigen Sammelbehälter.

Die flachen Wandungsabschnitte in definierter Ausrichtung verbessern das Verhalten erheblich. Durch das höhere Trägheitsmoment gelangt der Flüssigkeitsspeicher mit geringerer Wahrscheinlichkeit in eine rotierende oder trudelnde Bewegung. Die Gefahr einer exzentrischen Anströmung durch den Luftstoß ist zudem verringert.

Es hat sich gezeigt, dass Flüssigkeitsspender erfindungsgemäßer Art mit zwei flachen Wandungsabschnitten am Flüssigkeitsspeicher statistisch erheblich häufiger als Flüssigkeitsspender mit rundem Flüssigkeitsspeicher den richtigen Sammelbehälter erreichen. Besonders begünstigt wird dies, wenn ein c_{W}-Wert des Flüssigkeitsspenders bezogen auf eine Anströmrichtung auf einen flachen Wandungsabschnitt des Flüssigkeitsspeichers mehr als 0,6 beträgt, vorzugsweise zwischen 0,6 und 1,2. Insbesondere ein c_{W}-Wert zwischen 0,7 und 0,9 scheint zu begünstigen, dass der Flüssigkeitsspender in den richtigen Sammelbehälter gelangt. Eine Möglichkeit, den c_{W}-Wert gezielt zu beeinflussen und damit die gezielte Flugbahnbeeinflussung zu verbessern, besteht darin, auf den flachen Wandungsabschnitten des Flüssigkeitsspeichers leichte Vertiefungen vorzusehen, beispielsweise in Art von Griffmulden. Solche konkaven Vertiefungen haben sich ungeachtet des konkreten damit erzielten c_{W}-Werts als vorteilhaft bei der Abfalltrennung erwiesen.

Es hat sich weiterhin gezeigt, dass vorteilhaft die Außenkontur eine Oberfläche mit einer mittleren Oberflächenrauheit Ra von weniger als 10 µm aufweist, insbesondere weniger als 2 µm. Höhere Oberflächenrauheiten scheinen das Verhalten des Flüssigkeitsspenders beim Beaufschlagen mit einem Luftstoß negativ zu beeinflussen.

Die Speicherwandung des Flüssigkeitsspeichers kann zumindest über einen überwiegenden Längenanteil der durch den Flüssigkeitsspeicher gebildeten Außenkontur zylindrisch ausgeprägt sein. Von besonderem Vorteil kann es jedoch auch sein, wenn stattdessen die Speicherwandung in den Randbereichen zwischen den flachen Wandungsabschnitten gewölbt ist, um noch weiter zu begünstigen, dass der Flüssigkeitsspender in flach liegender Position auf dem Förderband transportiert wird. Eine Wölbung im besagten Randbereich verringert eine seitliche Auflagefläche und damit die Restgefahr, dass der Flüssigkeitsspender auf dem Randbereich stehend auf dem Förderband transportiert wird.

Der Querschnitt der Speicherwandung ist vorzugsweise im Wesentlichen oval oder elliptisch ausgebildet. Auch dies trägt mit hoher Wahrscheinlichkeit dazu bei, dass der Flüssigkeitsspender in der definierten liegenden Position auf dem Förderband zugeführt wird. Insbesondere kann der Querschnitt der Speicherwandungein Breite-Tiefe-Verhältnis von 1,2 oder mehr aufweisen, vorzugsweise von 1,5 oder mehr.

Es hat sich weiterhin gezeigt, dass auch bei definierter liegender Stellung bei der Zuführung des Flüssigkeitsspenders auf dem Förderband Unterschiede in der Qualität der Trennung in Abhängigkeit von der Form und der Gewichtsverteilung des Flüssigkeitsspenders festzustellen sind.

Die nachfolgenden Ausführungen nehmen Bezug auf eine Silhouette der Außenkontur des Flüssigkeitsspenders. Es handelt sich hierbei um die Silhouette der Außenkontur in Draufsicht des flach auf dem Förderband liegenden Flüssigkeitsspenders.

Es ist bevorzugt, dass bei der genannten Silhouette ein durch Oberflächen des Flüssigkeitsspeicher gebildeter Teil der Außenkontur einen größeren Flächenanteil einnimmt als ein durch den Austragkopf gebildeter Teil. Der Austragkopf ist üblicherweise schmaler als der Flüssigkeitsspeicher und bildet daher einen weniger geeigneten Teil des Flüssigkeitsspenders für die Anströmung durch einen Luftstoß.

Wenn der größere Flächenanteil durch den Flüssigkeitsspeicher gebildet wird, so erfolgt in den genannten Recycling-Anlagen auch die Anströmung statistisch häufiger im Bereich des Flüssigkeitsspeichers und damit am flachen Wandungsabschnitt, der sich für die Anströmung und die Flugbahnbeeinflussung besonders eignet. Bevorzugt ist es, wenn das Verhältnis der beiden Flächenanteile vorzugsweise zwischen 1,1:1 und 2:1, insbesondere zwischen 1,2:1 und 1,5:1, beträgt.

Weiterhin hat es sich als vorteilhaft herausgestellt, wenn der durch den Flüssigkeitsspeicher gebildete Flächenanteil der Silhouette eine Höhe und Breite aufweist, die nicht weit voneinander abweichen. Ein Quotient aus der Höhe des durch den Flüssigkeitsspeicher gebildeten Teils in Haupterstreckungsrichtung und der Breite des durch den Flüssigkeitsspeicher gebildeten Teils quer zur Haupterstreckungsrichtung beträgt vorzugsweise zwischen 0,8 und 1,2, insbesondere zwischen 0,9 und 1,1. Der Flächenanteil des Flüssigkeitsspeichers bildet grob die Form eines Quadrats oder eines recht kurzen Rechtecks. Dies begünstigt bei der Analyse durch die Steuereinheit der Recycling-Anlage die Identifikation dieses Flächenanteils des Flüssigkeitsspeichers als Anströmfläche für den Luftstoß.

Bezogen auf die genannte Silhouette in flach liegender Ausrichtung des Flüssigkeitsspenders ist ein Flächenschwerpunkt der Silhouette vorzugsweise am Übergang zwischen dem Austragkopf und dem Flüssigkeitsspeicher vorgesehen oder unterhalb dessen im Bereich des Flüssigkeitsspeichers, aber vorzugsweise nicht weiter als 15 mm, vorzugsweise nicht weiter als 10 mm, vom Übergang in Richtung des Flüssigkeitsspeichers entfernt.

Der Flächenschwerpunkt ist somit am oberen Ende des durch den Flüssigkeitsspeicher gebildeten Flächenanteils der Silhouette. Es hat sich herausgestellt, dass dies eine nahezu ideale Anströmung durch den Luftstoß zur Folge hat. Besonders bevorzugt ist ein Flächenschwerpunkt der Silhouette etwa 2 mm bis 8 mm unterhalb des Übergangs zwischen Austragkopf und Flüssigkeitsspeicher.

Der Austragkopf ist bei einem erfindungsgemäßen Spender vorzugsweise deutlich schmaler als der Flüssigkeitsspeicher quer zur Haupterstreckungsrichtung. Ein Quotient aus der Breite des Austragkopfes quer zur Haupterstreckungsrichtung des Flüssigkeitsspenders und der maximalen Breite des Flüssigkeitsspeichers quer zur Haupterstreckungsrichtung beträgt weniger als 0,8, vorzugsweise weniger als 0,7. Dies bezieht sich vorzugsweise insbesondere auf eine Situation mit fehlender Schutzkappe, wie sie im Rahmen der Abfallverwertung nicht unüblich ist.

Bezogen auf die Haupterstreckungsrichtung ist die Länge des durch den Austragkopf gebildeten Teils der Außenkontur bei den Spendertypen, bei denen eine erfindungsgemäße Ausgestaltung als besonders hilfreich angesehen wird, üblicherweise größer als die Länge des durch den Flüssigkeitsspeicher gebildeten Teils der Außenkontur. Üblich ist hier insbesondere ein Faktor der Länge des Austragkopfes zur Länge des Flüssigkeitsspeichers in Haupterstreckungsrichtung zwischen 1,2 und 1,8, insbesondere zwischen 1,4 und 1,7.

Die schmalere und ggf. längliche Gestaltung des Austragkopfes verglichen mit dem Flüssigkeitsspeicher begünstigt ebenfalls, dass die Recycling-Anlage den Flüssigkeitsspeicher als Anströmfläche für den Luftstoß auswählt und somit den Flüssigkeitsspender in den richtigen Sammelbehälter befördert.

Auch ist es von Vorteil, wenn bezogen auf die genannte Silhouette die Lage des Flächenschwerpunktes und des Massenschwerpunktes des entleerten Flüssigkeitsspeichers nicht weit voneinander entfernt sind. Es ist anzustreben, dass der Flächenschwerpunkt und der Massenschwerpunkt bezogen auf die Silhouette um weniger als 20 mm voneinander beabstandet sind, insbesondere um weniger als 15 mm. Ein geringer Abstand zwischen Flächenschwerpunkt und Massenschwerpunkt verringert die Gefahr einer trudelnden Bewegung in Folge des Luftstoßes.

Besonders vorteilhaft wird eine Bauweise angesehen, bei der sowohl der Massenschwerpunkt als auch der Flächenschwerpunkt bezogen auf die Silhouette unterhalb eines Übergangs zwischen dem Austragkopf und dem Flüssigkeitsspeicher vorgesehen sind und sich also im Bereich des Flüssigkeitsspeichers befinden.

Die genannten Maximalabstände zwischen Massenschwerpunkt und Flächenschwerpunkt beziehen sich vorzugsweise auf einen Zustand des vollständig entleerten Flüssigkeitsspenders mit und ohne aufgesetzte Schutzkappe, insbesondere jedoch auf den Zustand ohne Schutzkappe.

Die Beeinflussung des Schwerpunktes im oben beschriebenen Sinne kann auf verschiedene Weise erfolgen. Da ein relativ tiefer Schwerpunkt angestrebt wird, kann insbesondere durch eine besonders leichte Bauweise des Austragkopfes oder eine vergleichsweise schwere Bauweise des Flüssigkeitsspeichers das gewünschte Ziel erreicht werden.

Die Erzielung eines tiefen Schwerpunktes kann insbesondere auch dadurch begünstigt werden, dass der Flüssigkeitsspeicher zumindest überwiegend aus dem vergleichsweise dichten Material Polyethylenterephthalat (PET) besteht und der Austragkopf zumindest zum überwiegenden Teil aus PE und/oder PP gefertigt ist.

Weiterhin kann es von Vorteil sein, wenn die vergleichsweise schwere Pumpeinrichtung zumindest teilweise durch eine Entnahmeöffnung des Flüssigkeitsspeichers in diesen hineinragt, um hierdurch einen tiefen Schwerpunkt zu erzielen.

Wie eingangs bereits beschrieben, ist ein erfindungsgemäßer Flüssigkeitsspender vollständig aus Kunststoff hergestellt, so dass insbesondere keinerlei metallische Bestandteile in den Recycling-Strom gelangen.

Im Falle einer Gestaltung des Flüssigkeitsspenders mit einer Pumpeinrichtung weist die Pumpeinrichtung vorzugsweise eine Rückstellfeder auf, die ebenfalls aus Kunststoff besteht.

Eine mögliche Bauweise sieht vor, dass diese Kunststofffeder intern und von außen unsichtbar Teil des Austragkopfes ist und vorzugsweise mit ihrem unteren Ende bis in den Flüssigkeitsspeicher hineinragt.

Es hat sich jedoch als besonders vorteilhaft herausgestellt, eine andere Bauweise zu wählen, bei der eine Balgfeder aus Kunststoff Verwendung findet, die außenseitig vorgesehen ist, so dass sie einen Teil der Außenkontur des Flüssigkeitsspenders bildet.

Insbesondere handelt es sich vorzugsweise um eine außenliegende Balgfeder mit einem Außendurchmesser von mindestens 15 mm, vorzugsweise von mindestens 18 mm. Weiterhin wird eine Länge von mindestens 10 mm und vorzugsweise mindestens 15 mm als ideal angesehen. Eine Oberflächenrauheit Ra von mindestens 3 µm wird im Bereich der Balgfeder als vorteilhaft angesehen, vorzugsweise von mindestens 10 µm.

Es hat sich gezeigt, dass die außenliegende Balgfeder eine begünstigende Wirkung auf die Anströmung durch einen Luftstoß hat. Flüssigkeitsspender mit außenliegender Balgfeder scheinen in geringerem Maße als Flüssigkeitsspender mit zylindrischer Ausprägung der Außenkontur am Austragkopf in eine rotierende oder trudelnde Bewegung zu geraten.

Ein erfindungsgemäßer Spender weist vorzugsweise eine Fingerauflage auf, die vom Benutzer zum Zwecke des Flüssigkeitsaustrags niedergedrückt wird.

Insbesondere kann diese Fingerauflage als umlaufende Fingerauflage oder in Form zweier radial zur Haupterstreckungsrichtung wegweisende Fingerauflageflügel ausgebildet sein.

Es wird bevorzugt, wenn die Fingerauflage eine nicht-runde Form aufweist, wobei eine maximale Erstreckung der Fingerauflage mit der Ebene der flach liegenden Ausrichtung des Flüssigkeitsspenders einen Winkel von weniger als 30° einschließt und vorzugsweise in der Ebene der flach liegenden Ausrichtung liegt. Die Fingerauflage ist also vorzugsweise korrespondierend mit der von oben gesehen länglichen Erstreckung des Flüssigkeitsspeichers ausgerichtet.

Insbesondere überragt die Fingerauflage den Flüssigkeitsspeicher vorzugsweise nicht, so dass sie in der flach liegenden Ausrichtung des Flüssigkeitsspenders keinen Kontakt mit einem ebenen Untergrund hat und somit die definierte Ausrichtung des Flüssigkeitsspenders auf dem Förderband nicht beeinflusst. Alternativ kann die Fingerauflage auch derart bemessen sein, dass sie bei liegender Ausrichtung des Flüssigkeitsspenders der beschriebenen Art in Kontakt mit dem Förderband steht, jedoch hierdurch die liegende Ausrichtung nicht stört, sondern im Gegenteil diese mitbewirkt und stützt.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 und 2 verdeutlichen den Grundaufbau einer Anlage zur Abfalltrennung im Zuge des Recyclings und den Prozess der automatischen Abfalltrennung.
Fig. 3 bis 8 zeigen einen exemplarischen Flüssigkeitsspender in Art eines Nasalspenders, der für die automatische Abfalltrennung optimiert ist.
Fig. 9A und 9B zeigen einen alternativen Flüssigkeitsspender mit einer in Hinblick auf den c_{W}-Wert optimierten Gestaltung des Flüssigkeitsspeichers.
Fig. 10A und 10B zeigen einen alternativen Flüssigkeitsspender mit einer in Hinblick auf die Gewährleistung einer liegenden Position auf einem Förderband der Anlage zur Abfalltrennung optimierten Formgebung des Flüssigkeitsspeichers.
Fig. 11 und 12 zeigen Variationen von Fingerauflagen an erfindungsgemäßen Flüssigkeitsspendern.
Fig. 13 und 14 zeigen erfindungsgemäße Flüssigkeitsspender mit anderen Anwendungsfeldern.

### DETAILLIERTE BESCHREIBUNG DER AUSFÜHRUNGSBEISPIELE

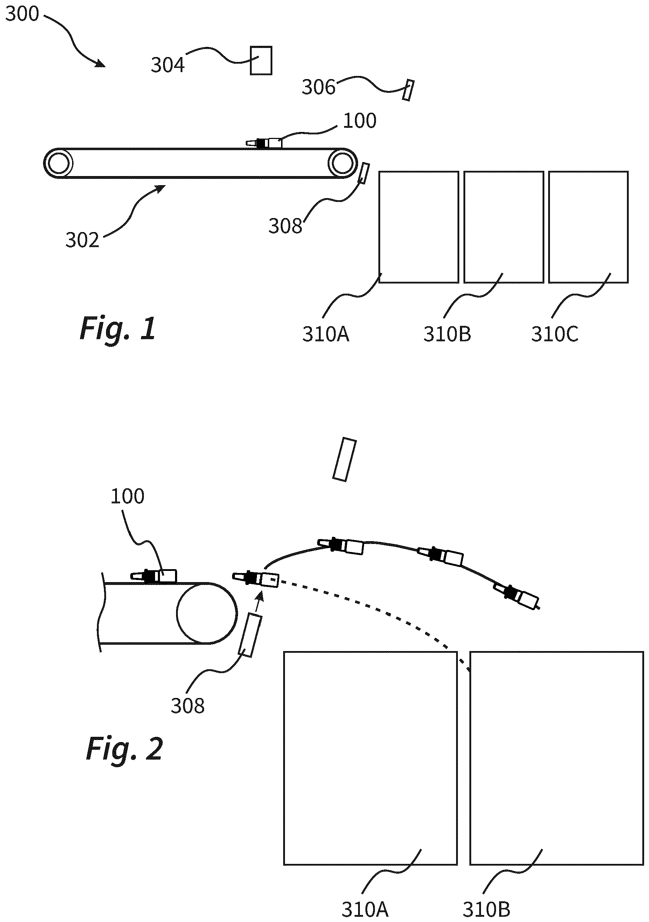

Fig. 1 veranschaulicht in vereinfachter Form ein modernes Trennverfahren im Rahmen des AbfallRecyclings mittels einer Trennanlage 300. Das Verfahren sieht vor, dass Abfall auf einem Förderband 302 zugeführt wird und je nach Art des Materials bestimmungsgemäß in einen von mehreren Sammelbehältern 310A, 310B, 310C gelangen sollen.

Um dies zu ermöglichen, ist eine Erfassungseinrichtung 304 vorgesehen, die oberhalb des Förderbandes 302 angeordnet ist und die auf dem Förderband 302 geförderte Abfallobjekten erfasst. Erfasst werden hierbei insbesondere der Kunststofftyp, beispielsweise mittels Infrarot-Scanners, sowie die Position des Abfallobjektes auf dem Förderband.

Abfallobjekte, die das Ende des Förderbandes 302 erreicht haben, fallen in Richtung der Sammelbehälter 310A, 310B, 310C, wobei diese Sammelbehälter 310A, 310B, 310C für unterschiedliche Abfallarten vorgesehen sind, insbesondere unterschiedliche Kunststofftypen. Vorliegend ist der Sammelbehälter 310B für Polyethylenterephthalat (PET) sowie Polyolefine vorgesehen.

Der auf dem Förderband 302 dargestellte Flüssigkeitsspender 100 besteht aus PET und PP und gehört daher in den Sammelbehälter 310B.

Teil der Trennanlage 300 sind zwei Düsenleisten 306, 308, die jeweils über eine Vielzahl einzelner Luftdüsen verfügen, die einzeln oder gruppenweise über Schaltventile geöffnet werden können, um einen Luftstoß zu erzeugen. Die Düsenleiste 306 ist oberhalb des Förderbandes 302 angeordnet und dafür vorgesehen, die Flugbahn von Objekten zu verkürzen, so dass diese in den Sammelbehälter 310A fallen. Die gegenüberliegende Düsenleiste 308 ist unterhalb des Förderbandes 302 angeordnet und kann durch einen nach oben gerichteten Luftstoß die Flugbahn von Objekten verlängern, so dass sie je nach Art des Luftstoßes in den Sammelbehälter 310B oder 310C fallen.

Fig. 2 verdeutlicht dies. Die gestrichelt dargestellte Bahn ist die ballistische Bahn, auf der Abfallobjekte, die das das Ende des Förderbandes 302 erreicht haben, hinabfallen würden. Sie würden damit in einen der beiden Sammelbehälter 310A und 310B fallen. Mittels der Düsenleiste 308 wird ein Flüssigkeitsspender 100 unmittelbar nach dem Ende des Förderbandes 302 mit einem Luftstoß beaufschlagt, so dass er wunschgemäß der durchgezogenen Flugbahn folgend im Sammelbehälter 310B landet.

Allerdings hat es sich in der Praxis als schwierig herausgestellt, die vergleichsweise kleinen Flüssigkeitsspender 100 ausreichend präzise mit einem Luftstrom zu beeinflussen, um zumindest nahezu 100% solcher Flüssigkeitsspender 100 im Sammelbehälter 310B sammeln zu können.

Die weiteren Figuren erläutern, wie der Flüssigkeitsspender 100 ausgebildet sein kann, um einen hohen Anteil von Flüssigkeitsspendern 100 erfindungsgemäßer Art im jeweils richtigen Sammelbehälter 310B sammeln zu können.

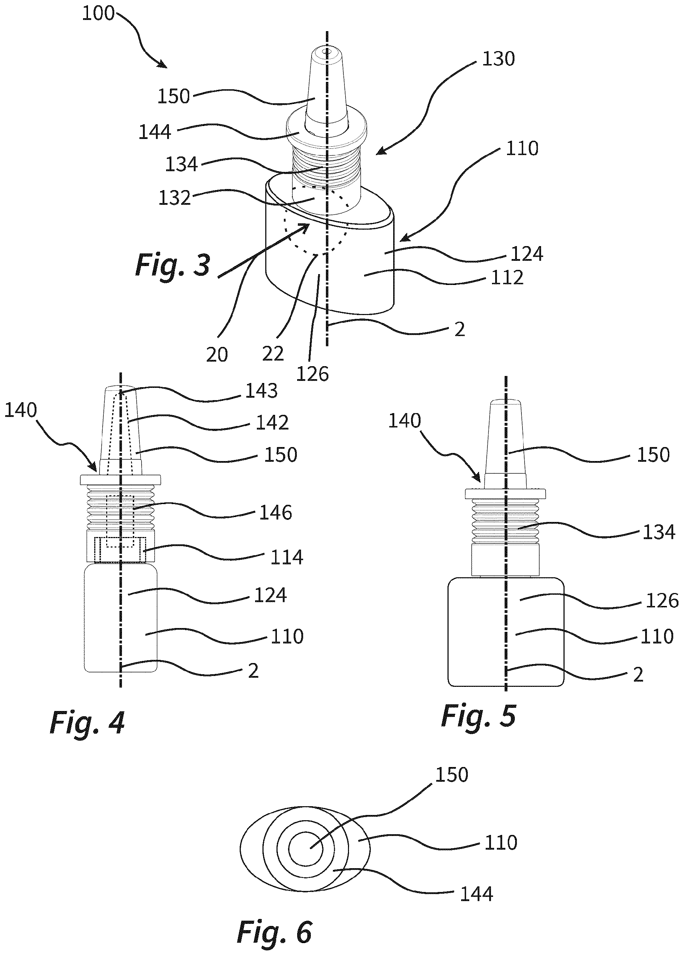

Die Fig. 3 bis 6 zeigen einen erfindungsgemäßen Flüssigkeitsspender 100. Der Flüssigkeitsspender 100 verfügt über einen Flüssigkeitsspeicher 110, der durch einen Hohlkörper aus Polyethylenterephthalat gebildet ist. Ein in Fig. 4 dargestellter Hals 114 des Flüssigkeitsspeichers ist außenseitig mit einem Gewinde versehen, welches in ein nicht näher dargestelltes Innengewinde an einem Austragkopf 130 eingeschraubt ist. Der Austragkopf 130 seinerseits verfügt über eine Basis 132, an der das genannten Innengewinde vorgesehen ist, sowie über eine Betätigungseinheit 140, die eine Fingerauflage 144 sowie einen länglichen Nasalapplikator 142 umfasst. Am distalen Ende des Nasalapplikators ist eine Austragöffnung 143 für die Abgabe von Flüssigkeit vorgesehen. Der Austragkopf 130 besteht vorzugsweise ausschließlich aus Polyethylen oder Polypropylen oder zum Teil aus Polyethylen und zum Teil aus Polypropylen oder Polyethylenterephthalat.

Die Betätigungseinheit 140 wird zum Zwecke des Austrags gegenüber der Basis 132 niedergedrückt. Hierdurch wird eine Pumpeinrichtung 146 betätigt, die in nicht näher dargestellter Weise über ein Einlassventil und ein Auslassventil verfügt und deren Pumpkammer bei einem Niederdrücken der Betätigungseinheit 140 verkleinert wird, so dass das Auslassventil geöffnet und Flüssigkeit durch die Austragöffnung 143 hindurch ausgetragen wird.

Zum Zwecke der Rückstellung der Betätigungseinheit 140 in die unbetätigte Stellung ist eine Rückstellfeder 134 vorgesehen, die als Balgfeder ausgestaltet ist und die außenseitig am Flüssigkeitsspender 100 angeordnet ist.

Wenn nach einem Austrag die manuelle Kraftbeaufschlagung der Fingerauflage 144 nach unten entfällt, drückt die Rückstellfeder 134 die Betätigungseinheit 140 wieder nach oben. Hierbei öffnet das Eingangsventil und Flüssigkeit aus dem Flüssigkeitsspeicher 110 wird in die Pumpkammer eingesogen. Wie sich anhand der Darstellung der Fig. 4 ergibt, ist die Pumpeinrichtung 146 vorzugsweise relativ tief angeordnet, insbesondere derart, dass ein Teil der Pumpkammer und/oder ein Teil des Einlassventils bis in den Hals 114 des Flüssigkeitsspeichers 110 hineinragen.

Neben der Tatsache, dass der Flüssigkeitsspender 100 vollständig aus Kunststoff besteht, liegt die Besonderheit des Spenders in seiner kompakten Bauweise. Der Flüssigkeitsspender weist in Richtung seiner Haupterstreckungsrichtung 2 eine Länge von unter 100 mm auf.

Weiterhin weist der Flüssigkeitsspender 100 erfindungsgemäß die Besonderheit auf, dass der Flüssigkeitsspeicher dafür optimiert ist, auf einer Recycling-Anlage entsprechend der Fig. 1 und 2 mit besonders hoher Wahrscheinlichkeit den richtigen Sammelbehälter zu erreichen.

Zu diesem Zweck ist der Flüssigkeitsspeicher mit einem ovalen Querschnitt vorgesehen. Die umlaufende Speicherwandung 112 weist also nicht die übliche Form einer Kreisform auf, sondern eine elliptische Form. Die maximale Breite beträgt etwa 150% der maximalen Tiefe.

Diese Formgebung ist primär aus zwei Gründen gewählt.

Zum einen wird hierdurch erreicht, dass der Flüssigkeitsspender auf dem Förderband 302 annähernd mit Sicherheit eine liegende Stellung einnimmt, in der die Ovalform des Flüssigkeitsspeichers 110 horizontal erstreckt ist. Dass der Flüssigkeitsspender 100 auf dem Förderband steht, ist durch die vergleichsweise geringe Tiefe des Flüssigkeitsspeichers 110 und den relativ hohen Schwerpunkt unwahrscheinlich. Dass der Flüssigkeitsspender eine liegende Stellung einnimmt, dabei jedoch auf einer Kante im Randbereich 124 liegt, ist ebenfalls in der Praxis unwahrscheinlich. Die ovale Form führt dazu, dass deutlich über 99% von Flüssigkeitsspendern der beschriebenen Art, die eine zufällige Ausrichtung auf dem Förderband einnehmen, die besagte liegende Stellung mit liegender OvalForm einnehmen, in der einer der beiden flachen Wandungsabschnitte 126 auf dem Förderband 302 aufliegt.

Zum anderen hat sich gezeigt, dass die flache Bauform des Flüssigkeitsspeichers 110, die sich durch den ovalen bzw. elliptischen Querschnitt ergibt, eine erheblich verbesserte Wahrscheinlichkeit zur Folge hat, dass der Flüssigkeitsspender 100 durch einen Luftstoß der Düsenleisten 306, 308 in den beabsichtigten Sammelbehälter 310B gelangt.

Der Luftstoß, der im vorliegenden Beispiel durch die untere Düsenleiste 308 erzeugt wird, trifft reproduzierbar auf einen der beiden flachen bzw. nur leichte Wandungsabschnitte 126 des Flüssigkeitsspenders 100. In Fig. 3 ist dies verdeutlicht. Der Pfeil 20 zeigt die Richtung des Luftstoßes, die gestrichelte Fläche 22 verdeutlicht die Zone, in der der Luftstoß primär auf den Flüssigkeitsspender 100 trifft.

Es hat sich gezeigt, dass diese Form der Anströmung abweichend von der Anströmung bei einem als Ganzes rotationssymmetrischen Spender eine reproduzierbare Veränderung der Flugbahn des Flüssigkeitsspenders 100 zur Folge hat. Selbst wenn der Luftstrom den Flüssigkeitsspeicher 110 nicht perfekt trifft, führt dennoch der weit überwiegende Teil der hiervon ausgehenden Energie in eine Änderung der Flugbahn des Flüssigkeitsspenders 100. Eine starke Rotation oder gar ein Trudeln des Flüssigkeitsspenders 100 in Folge des Luftstoßes ist jedoch nicht beobachtet worden.

Es wurde ermittelt, dass ein c_{W}-Wert des Flüssigkeitsspenders 100 bezogen auf die seitliche Anströmrichtung orthogonal zur Haupterstreckungsrichtung 2 zwischen 0,6 und 1,2 vorteilhaft ist. Vorliegend beträgt der Cw-Wert etwa 0,7.

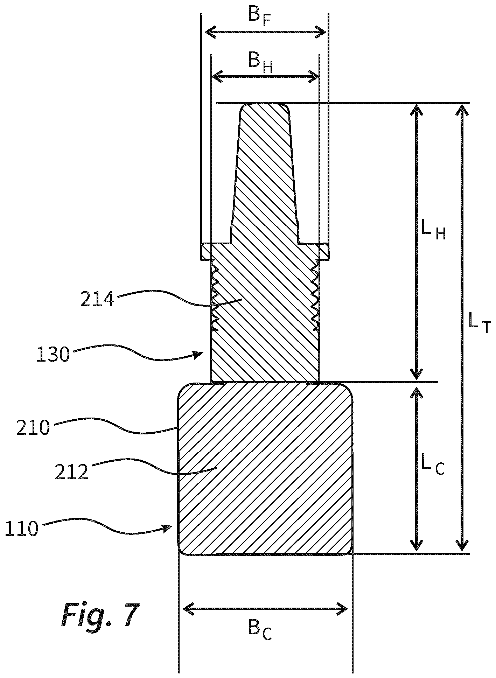

In Fig. 7 ist die Silhouette 210 des Flüssigkeitsspenders 100 bezogen auf dessen liegende Ausrichtung auf dem Förderband 302 in einer Darstellung von oben gezeigt, also jene Silhouette 210, die auch von der Erfassungseinrichtung 304 erfasst wird, wenn diese das Objekt auf dem Förderband 302 erfasst und weiterverarbeitet.

Der Flüssigkeitsspender gemäß diesem Ausführungsbeispiel hat eine Gesamtlänge von LT = 92 mm in Haupterstreckungsrichtung bei aufgesetzter Schutzkappe 150. Der Flüssigkeitsspeicher 110 bildet mit seinem angeströmten flachen Wandungsabschnitt eine nahezu quadratische Fläche einen und einen Teil 212 der Silhouette 210 mit einer Höhe von L_{C} = 35 mm und einer Breite von B_{C} = 35 mm. Der Austragkopf 130 einschließlich der Schutzkappe 150 weist in Haupterstreckungsrichtung 2 eine Länge von L_{H} = 37 mm auf. Die Breite B_{H} des Austragkopfes 130 im Bereich der Balgfeder 134 beträgt 19 mm. Der Durchmesser B_{F} im Bereich der Fingerauflage 144 beträgt 23 mm.

Bei diesen Maßen bildet der Flüssigkeitsspeicher mit seiner nur leicht gewölbten Anströmfläche den überwiegenden Teil 212 der Silhouette, im konkreten Beispiel etwa 57% der Gesamtfläche der Silhouette 210. Der Austragkopf bildet lediglich einen etwa 43% der Gesamtgröße der Silhouette 210 bildenden Teil 214.

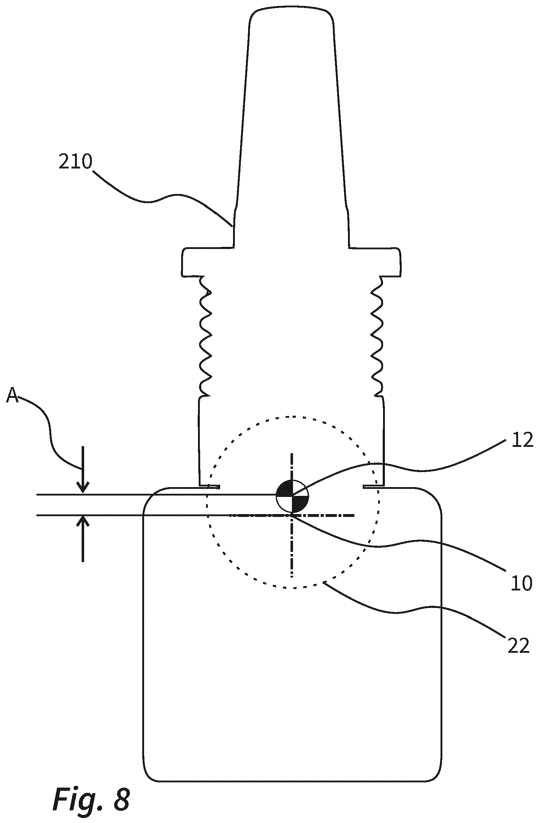

Fig. 8 verdeutlicht die Lage des Flächenschwerpunktes 10 relativ zum Massenschwerpunkt 12.

Der Flächenschwerpunkt 10 ist etwa 3 mm bis 5 mm unterhalb eines Übergangs zwischen dem Flüssigkeitsspeicher 110 und dem Austragkopf 130 zu finden. Der Flächenschwerpunkt 10 ist jene Stelle, die im statistischen Mittel durch Recycling-Anlagen wie jeder der Fig. 1 und 2 mittels des Luftstoßes getroffen wird.

Der Massenschwerpunkt 12 ist idealerweise dem Flächenschwerpunkt 10 möglichst nahe. Da der Flüssigkeitsspeicher 110 nach Verbrauch der Flüssigkeit hohl ist und der Austragkopf 130 mit der Pumpeinrichtung 146 vergleichsweise schwer ist, ist der Massenschwerpunkt 12 üblicherweise oberhalb des Flächenschwerpunktes 10 zu finden. Vorliegend ist durch Verwendung des besonders dichten Materials PET als Material des Flüssigkeitsspeichers 110 und Verwendung von PE mit deutlich geringerer Dichte als überwiegendes Material des Austragkopfes 130 jedoch erzielt worden, dass sich der Massenschwerpunkt 12 nur etwa 3 mm oberhalb des Flächenschwerpunktes 10 befindet.

Dies begünstigt zusätzlich, dass der Flüssigkeitsspender 100 beim Anströmen mit einem Luftstoß der Düsenleiste 308 nicht in eine Rotationsbewegung oder ein Trudeln gerät.

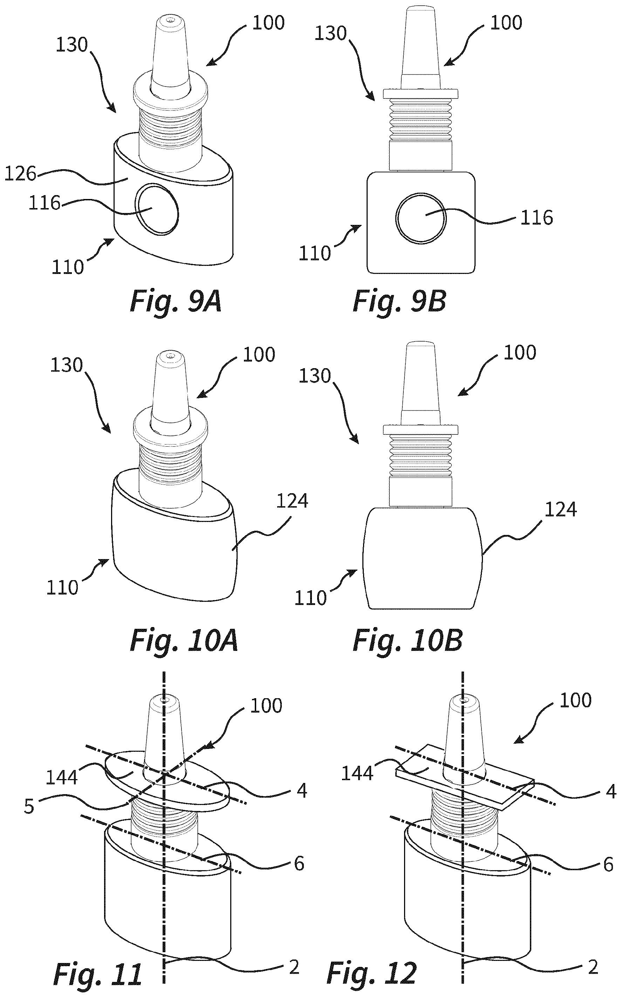

Die Fig. 9A bis 14 zeigen weitere vorteilhafte Gestaltungmerkmale und Variationen des Flüssigkeitsspenders 100.

Die Gestaltung der Fig. 9A und 9B weist im Bereich des flachen Wandungsabschnitts 126 an der Vorderseite und ebenso an der Rückseite im Bereich der Anströmfläche eine Vertiefung 116 auf. Diese Vertiefung 116 führt zu einer Erhöhung des c_{W}-Wertes. Über eine solche Vertiefung und ihre konkreten Maße lässt sich der c_{W}-Wert eines Flüssigkeitsspenders 100 bei im übrigen gleichbleibender Formgebung sehr gut beeinflussen, um ein optimales Verhalten bei der Recycling-Trennung zu erzielen.

Fig. 10A und 10B zeigen eine Variante, bei der die schmalen Randbereiche 124 des Flüssigkeitsspeichers 110 mit einer nach außen gerichteten Wölbung versehen sind. Eine solche Wölbung kann vorteilhaft sein, um die Wahrscheinlichkeit nochmals weiter zu reduzieren, dass der Flüssigkeitsspender 100 auf dem Förderband 302 auf dem Randbereich 124 zum Liegen kommt und nicht die gewünschte flach liegende Ausrichtung einnimmt.

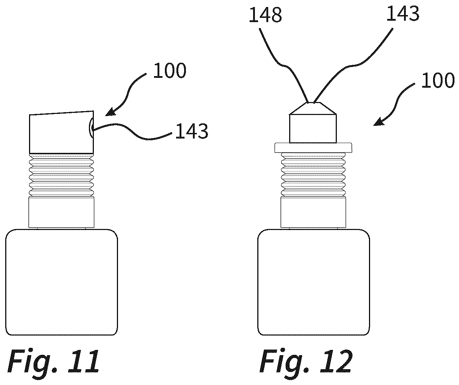

Fig. 11 und 12 zeigen Bauweisen des Flüssigkeitsspenders 100, bei denen die Haupterstreckungsrichtungen 4 der Fingerauflagen 144 mit der der größten Erstreckungsrichtung 6 der Flüssigkeitsspeicher 110 quer zur Haupterstreckungsrichtung 2 übereinstimmen. Die Haupterstreckungsrichtungen 4 der Fingerauflagen 144 sind somit in etwa parallel zu den flachen Wandungsabschnitten 126 ausgerichtet, so dass sie die gewünschte flach liegende Stellung des Flüssigkeitsspenders 100 auf dem Förderband 302 nicht stören.

Im Falle der Gestaltung der Fig. 11 entspricht die Erstreckung der Fingerauflage 144 in einer Richtung 5 quer zur Haupterstreckungsrichtung 4 der Fingerauflage 144 der minimalen Erstreckung des Flüssigkeitsspeichers 110 quer zur Haupterstreckungsrichtung 2 des Flüssigkeitsspeichers 110. Die Fingerauflage 144 stützt daher zusätzlich die gewünschte flach liegende Stellung des Flüssigkeitsspenders 100 auf dem Förderband 302.

Fig. 13 und 14 verdeutlichen alternative Bauweisen für anderweitige Zwecke. Während die Flüssigkeitsspender der Fig. 3 bis 9 als Nasalspenders ausgebildet sind, ist der Flüssigkeitsspender 100 der Fig. 12 als topischer Spender gestaltet. Seine Austragöffnung 143 ist daher seitlich angeordnet. Die Oberseite der Betätigungseinheit 140 bildet eine Fingerauflagefläche. Fig. 13 zeigt einen als Tropfenspender ausgebildeten Flüssigkeitsspender 100. Dieser verfügt über eine Austragöffnung 143, die von einer Tropfenbildungsfläche 148 umgeben ist. Während der Betätigung in Überkopflage wird Flüssigkeit unter geringem Druck abgegeben, die sich an der Tropfenbildungsfläche 148 ansammelt und sich bei Erreichen eines ausreichenden Tropfenvolumens löst.

## Patentansprüche

1. Medizinischer Flüssigkeitsspender (100) mit den folgenden weiteren Merkmalen:
a. der Flüssigkeitsspender (100) verfügt über einen Flüssigkeitsspeicher (110) und einen am Flüssigkeitsspeicher (110) angebrachten Austragkopf (130), deren jeweilige Außenseite einen Teil einer Außenkontur des Flüssigkeitsspenders (100) bilden, und
b. der Flüssigkeitsspender (100) weist eine Länge (L_{T}) in einer Haupterstreckungsrichtung (2) von weniger als 125 mm und eine Leermasse von weniger als 20 Gramm auf, und
c. der Flüssigkeitsspender (100) besteht ausschließlich aus Kunststoffmaterialien, **gekennzeichnet durch** das folgende zusätzliche Merkmal:
d. zur verbesserten Separierung im Rahmen des Abfallrecyclings ist ein Querschnitt des Flüssigkeitsspeichers (110) flach ausgebildet, so dass eine liegende Position auf einem Recyclingförderband (302) in einer von zwei liegenden Ausrichtungen begünstigt wird.

2. Medizinischer Flüssigkeitsspender (100) nach Anspruch 1 mit dem folgenden weiteren Merkmal:
a. ein Querschnitt einer Speicherwandung (112) des Flüssigkeitsspeichers (110) weist eine im Wesentlichen ovale oder elliptische Form auf,
vorzugsweise mit dem folgenden zusätzlichen Merkmal:
b. der Querschnitt der Speicherwandung (112) weist ein Breite-Tiefe-Verhältnis von 1,2 oder mehr auf, vorzugsweise von 1,5 oder mehr.

3. Medizinischer Flüssigkeitsspender (100) nach Anspruch 1 oder 2 mit dem folgenden weiteren Merkmal:
a. bezogen auf eine Silhouette (210) der Außenkontur in Draufsicht in flach liegender Ausrichtung bildet ein durch den Flüssigkeitsspeicher (110) gebildeter Teil (212) einen größeren Flächenanteil als ein durch den Austragkopf (130) gebildeter Teil (214),
vorzugsweise mit dem folgenden zusätzlichen Merkmal:
b. das Verhältnis beträgt vorzugsweise zwischen 1,1:1 und 2:1, vorzugsweise 1,2:1 und 1,5:1.

4. Medizinischer Flüssigkeitsspender (100) nach einem der vorstehenden Ansprüche mit dem folgenden weiteren Merkmal:
a. bezogen auf eine Silhouette (210) der Außenkontur in Draufsicht in flach liegender Ausrichtung beträgt ein Quotient aus der Höhe (L_{C}) des durch den Flüssigkeitsspeicher (110) gebildeten Teils (212) und der Breite (B_{C}) des durch den Flüssigkeitsspeicher (110) gebildeten Teils (212) zwischen 0,8 und 1,2, vorzugsweise zwischen 0,9 und 1,1.

5. Medizinischer Flüssigkeitsspender (100) nach einem der vorstehenden Ansprüche mit dem folgenden weiteren Merkmal:
a. ein Quotient aus der maximalen Breite des Austragkopfes (B_{F}) quer zur Haupterstreckungsrichtung (2) des Flüssigkeitsspenders (100) und der maximalen Breite (B_{C}) des Flüssigkeitsspeichers (110) quer zur Haupterstreckungsrichtung (2) beträgt weniger als 0,8, vorzugsweise weniger als 0,7.

6. Medizinischer Flüssigkeitsspender (100) nach Anspruch 5 mit dem folgenden weiteren Merkmal:
a. bezogen auf die Haupterstreckungsrichtung (2) ist die Länge (L_{H}) des durch den Austragkopf (130) gebildeten Teils der Außenkontur größer als die Länge (L_{C}) des durch den Flüssigkeitsspeicher (110) gebildeten Teils der Außenkontur,
vorzugsweise mit dem folgenden zusätzlichen Merkmal:
b. ein Faktor der Länge (L_{H}) des Austragkopfes (130) zur Länge (L_{C}) des Flüssigkeitsspeichers (110) beträgt zwischen 1,2 und 1,8, vorzugsweise zwischen 1,4 und 1,7.

7. Medizinischer Flüssigkeitsspender (100) nach einem der vorstehenden Ansprüche mit dem folgenden weiteren Merkmal:
a. bezogen auf eine Silhouette (210) der Außenkontur in Draufsicht in flach liegender Ausrichtung ist ein Flächenschwerpunkt (10) am Übergang zwischen dem Austragkopf (130) und dem Flüssigkeitsspeicher (110) vorgesehen oder nicht weiter als 15 mm von hier in Richtung des Flüssigkeitsspeichers (110) entfernt,
vorzugsweise mit dem folgenden zusätzlichen Merkmal:
b. bezogen auf eine Silhouette (210) der Außenkontur in Draufsicht in flach liegender Ausrichtung ist der Flächenschwerpunkt (10) am Übergang zwischen dem Austragkopf (130) und dem Flüssigkeitsspeicher (110) vorgesehen oder nicht weiter als 10 mm von hier entfernt.

8. Medizinischer Flüssigkeitsspender (100) nach einem der vorstehenden Ansprüche mit dem folgenden weiteren Merkmal:
a. bezogen auf eine Silhouette (210) der Außenkontur in Draufsicht in flach liegender Ausrichtung ist ein Flächenschwerpunkt (10) bezogen auf die Haupterstreckungsrichtung (2) um einen Abstand (A) von maximal 20 mm beabstandet vom Massenschwerpunkt (12) des Flüssigkeitsspenders (100), vorzugsweise um einen Abstand A von maximal 15 mm.

9. Medizinischer Flüssigkeitsspender (100) nach einem der vorstehenden Ansprüche mit dem folgenden weiteren Merkmal:
a. ein c_{W}-Wert des Flüssigkeitsspenders (100) bezogen auf eine Anströmrichtung auf eine flache Seite des Flüssigkeitsspeichers (110) beträgt 0,6 oder mehr, vorzugsweise zwischen 0,6 und 1,2, insbesondere vorzugsweise zwischen 0,7 und 0,9.

10. Medizinischer Flüssigkeitsspender (100) nach einem der vorstehenden Ansprüche mit dem folgenden weiteren Merkmal:
a. die Außenkontur wird zumindest teilweise durch eine Rückstellfeder (134) einer Pumpeinrichtung (146) gebildet, die als Balgfeder (134) ausgestaltet ist,
vorzugsweise mit einem der folgenden zusätzlichen Merkmale:
b. die Balgfeder (134) weist einen Außendurchmesser von mindestens 15 mm auf, vorzugsweise von mindestens 18 mm, und/oder
c. die Balgfeder (134) weist eine Länge von mindestens 10 mm, vorzugsweise von 15 mm, auf.

11. Medizinischer Flüssigkeitsspender (100) nach einem der vorstehenden Ansprüche mit dem folgenden weiteren Merkmal:
a. eine Außenkontur des Flüssigkeitsspenders (100) wird zumindest teilweise durch eine flächige Fingerauflage (144) gebildet,
vorzugsweise mit einem der folgenden zusätzlichen Merkmale:
b. die Fingerauflage (144) weist eine nicht-runde Form auf, wobei ein maximaler Durchmesser der Fingerauflage (144) mit der Ebene der flach liegenden Ausrichtung des Flüssigkeitsspenders (100) einen Winkel von weniger als 30° einschließt, wobei vorzugsweise der maximale Durchmesser in der Ebene der flach liegenden Ausrichtung des Flüssigkeitsspenders (100) liegt, und/oder
c. die Fingerauflage (144) überragt den Flüssigkeitsspeicher (110) im Bereich der Speicherwandung nicht, so dass sie in der flach liegenden Ausrichtung des Flüssigkeitsspenders (100) keinen Kontakt mit einem ebenen Untergrund hat, oder
d. die Fingerauflage (144) ist derart an die Formgebung des Flüssigkeitsspeicher (110) angepasst, dass der Flüssigkeitsspender in liegender Ausrichtung auf dem Flüssigkeitsspeicher (110) und der Fingerauflage (144) aufliegt und die Haupterstreckungsrichtung (2) des Flüssigkeitsspenders (100) horizontal ausgerichtet ist.

12. Medizinischer Flüssigkeitsspender (100) nach einem der vorstehenden Ansprüche mit dem folgenden weiteren Merkmal:
a. die Außenkontur des Flüssigkeitsspenders (100) weist eine Oberfläche mit einer mittleren Oberflächenrauheit Rₐ von weniger als 10 µm auf, insbesondere weniger als 2 µm,
vorzugsweise mit dem folgenden zusätzlichen Merkmal:
b. die Außenkontur weist zumindest teilweise und vorzugsweise im Bereich einer Rückstellfeder (134) eine mittlere Oberflächenrauheit Rₐ von mindestens3 µm auf, vorzugsweise von mindestens 10 µm.

13. Medizinischer Flüssigkeitsspender (100) nach einem der vorstehenden Ansprüche mit dem folgenden weiteren Merkmal:
a. der Flüssigkeitsspender (100) besteht ausschließlich aus Polyethylen oder ausschließlich aus Polypropylen.

14. Medizinischer Flüssigkeitsspender (100) nach einem der Ansprüche 1 bis 12 mit dem folgenden weiteren Merkmal:
a. der Flüssigkeitsspender (100) besteht ausschließlich aus Polyethylenterephthalat sowie Polyethylen oder Polypropylen,
vorzugsweise mit dem folgenden zusätzlichen Merkmal
b. der Flüssigkeitsspeicher (110) besteht aus Polyethylenterephthalat.

15. Medizinischer Flüssigkeitsspender (100) nach einem der vorstehenden Ansprüche mit mindestens einem der folgenden zusätzlichen Merkmale:
a. der Flüssigkeitsspender (100) weist eine Länge (L_{T}) von weniger als 100 mm auf und/oder eine Masse von weniger als 15 Gramm, und/oder
b. der Flüssigkeitsspender (100) ist als Nasalspender ausgebildet und verfügt am Austragkopf (130) über einen länglichen Nasalapplikator (142), an dessen distalem Ende eine Austragöffnung vorgesehen ist, und/oder
c. der Flüssigkeitsspender (100) ist als Tropfenspender ausgebildet und verfügt am Austragkopf (130) über eine Tropfenbildungsfläche stromabwärts einer Austragsöffnung, und/oder
d. der Flüssigkeitsspender (100) ist als topischer Spender ausgebildet, und/oder
e. der Austragkopf (130) ist mittels einer Schnappverbindung oder einer Gewindeverbindung am Flüssigkeitsspeicher (110) angebracht.
